Europäisches Patentamt

(19) European Patent Office

Office Européen des brevets

(11) Numéro de publication : **0 268 533 B1**

(12) **FASCICULE DE BREVET EUROPEEN**

(45) Date de publication du fascicule du brevet :
02.01.91 Bulletin 91/01

(51) Int. Cl.$^5$ : **A23K 1/00**, A23K 1/18,
A61K 9/30, A61K 9/52

(21) Numéro de dépôt : 87402574.5

(22) Date de dépôt : 16.11.87

(54) **Nouvelle composition pour l'alimentation des ruminants contenant une substance biologiquement active et sa préparation.**

(30) Priorité : 17.11.86 FR 8615930

(43) Date de publication de la demande :
25.05.88 Bulletin 88/21

(45) Mention de la délivrance du brevet :
02.01.91 Bulletin 91/01

(84) Etats contractants désignés :
AT BE CH DE ES FR GB IT LI LU NL SE

(56) Documents cités :
EP-A- 0 188 953
EP-A- 0 206 890
WO-A-84/00282
WO-A-84/04657
WO-A-87/03172
FR-A- 2 401 619
FR-A- 2 401 620
FR-A- 2 565 101
US-H- 100 404

(73) Titulaire : RHONE-POULENC SANTE
20, avenue Raymond Aron
F-92160 Antony (FR)

(72) Inventeur : Autant, Pierre
14 Rue de Pourcheroux
F-03600 Commentry (FR)
Inventeur : Bourrain, Paul
32/0 Chemin des Peupliers
F-69570 Dardilly (FR)
Inventeur : Cartillier, André
Champfromenteau
F-03600 Commentry (FR)

(74) Mandataire : Le Pennec, Magali et al
RHONE-POULENC SANTE, Service Brevets,
20 Avenue Raymond Aron
F-92165 Antony Cédex (FR)

## Description

La présente invention concerne une nouvelle composition pour l'alimentation des ruminants, contenant une substance biologiquement active hydrosoluble, qui est stable dans un milieu dont le pH est égal ou supérieur à 5,5 et qui permet la libération de la substance active dans un milieu dont le pH est égal ou inférieur à 3,5.

Lorsqu'on administre à des ruminants certaines substances biologiquement actives (médicaments, aliments enrichis), il se produit lors du passage dans le rumen une destruction enzymatique de ces substances favorisée par le temps de séjour (quelques heures à plusieurs jours) et par le pH (compris entre 5 et 6).

Il importe donc de protéger ces substances biologiquement actives par des enrobages qui soient stables dans la panse des ruminants, qui résistent à la dégradation par les microorganismes et qui permettent la libération des substances biologiquement actives dans une partie de l'appareil digestif, plus particulièrement dans la caillette, dont le pH est inférieur ou égal à 3,5. Alors que la durée de protection dans la panse doit être relativement longue (plusieurs heures à quelques jours), la libération de la substance active dans la caillette doit s'effectuer en un temps relativement court (de quelques minutes à 1 ou 2 heures).

De nombreuses compositions d'enrobage ont été décrites par exemple dans les brevets français FR 78 23966 (2 401 620), FR 78 23968 (2 401 621) ou FR 81 18954 (2 514 261). Cependant, si ces compositions donnent des résultats satisfaisants lorsqu'elles sont utilisées pour enrober des substances relativement peu solubles dans l'eau (par exemple la méthionine dont la solubilité est voisine de 40 g/litre), elles ne sont pas parfaitement adaptées à l'enrobage de substances dont la solubilité dans l'eau est plus élevée (par exemple, le chlorhydrate de lysine dont la solubilité est voisine de 400 g/litre).

Il a maintenant été trouvé, et c'est ce qui fait l'objet de la présente invention, qu'une composition comprenant :
- un noyau constitué d'une substance active hydrosoluble ou d'une composition contenant une substance active hydrosoluble, ayant éventuellement subi un traitement de surface,
- une première couche d'enrobage (pré-enrobage) constituée d'une substance ou d'une composition sensible aux variations du pH, éventuellement associée à une charge minérale, et
- une deuxième couche d'enrobage (sur-enrobage) constituée d'une substance ou d'une composition hydrophobe éventuellement associée à une charge minérale,
est stable dans un milieu dont le pH est égal ou supérieur à 5,5 et permet la libération de la substance active dans un milieu dont le pH est égal ou inférieur à 3,5,
étant entendu que
- lorsque le noyau subi un traitement de surface, la couche de pré-enrobage et la couche de sur-enrobage ne contiennent pas nécessairement de charge minérale associée, et que,
- lorsque le noyau n'a pas subi de traitement de surface, la couche de pré-enrobage et éventuellement la couche de sur-enrobage contiennent une charge minérale associée.

Le traitement de surface des granulés à enrober consiste à pulvériser à la surface des granulés une solution d'un liant de nature hydrophobe contenant éventuellement une dispersion d'une charge minérale de nature plutôt hydrophile.

Le liant est de préférence choisi parmi les polymères hydrophobes tels que la zéine, l'éthylcellulose ou l'acétobutyrate de cellulose. Le liant peut être également constitué d'un polymère sensible aux variations du pH tel qu'un copolymère du styrène avec les vinylpyridines éventuellement en combinaison avec une substance hydrophobe telle qu'un acide gras, un ester gras ou un alcool gras.

Les charges minérales sont choisies plus particulièrement parmi les silices pyrogénées ou précipitées, comme par exemple l'Aerosil 300 et la lévilite, la carbonate de calcium ou le kaolin.

La stabilité à pH = 6 du granulé traité en surface augmentant avec la teneur en liant, il n'existe pas, théoriquement, de limite supérieure à la teneur en liant. Cependant, pour obtenir un granulé fini dont la teneur en substance biologiquement active est suffisante, il est particulièrement avantageux de limiter la teneur en liant au voisinage de 10 % du poids du granulé à enrober.

Généralement, la charge minérale hydrophile représente jusqu'à 10 % en poids du granulé à enrober, une teneur supérieure n'entraînant pas une augmentation significative de la stabilité à pH = 6.

La couche de pré-enrobage est généralement constituée d'un polymère basique filmogène sensible aux variations du pH éventuellement en association avec un polymère non hydrosoluble et/ou une substance hydrophobe, dans lequel est éventuellement dispersée une charge minérale lamellaire ou pulvérulente hydrophobe.

Parmi les polymères aminés basiques peuvent être cités les polymères contenant au moins un groupement amino basique et dont la teneur en azote basique est comprise entre 2 et 14 % tels que les dérivés aminés de la cellulose, les polymères et copolymères des dérivés aminés des acides acrylique, méthacrylique et crotonique et les polymères ou copolymères du styrène ou de l'acrylonitrile avec les isomères ou les dérivés de la vinylpyridine tels que la vinyl-2 pyridine, la vinyl-4 pyridine ou la méthyl-2 vinyl-5 pyridine. D'un intérêt tout particulier sont les copolymères du styrène avec les isomères ou les dérivés de la vinylpyridine et plus spécialement les copolymères du styrène avec la vinyl-2 pyridine.

Le polymère non hydrosoluble est généralement choisi parmi les dérivés de la cellulose tels que l'éthyl-cellulose, l'acétobutyrate de cellulose et le propionate de cellulose, ou la zéine.

Les substances hydrophobes sont généralement choisies parmi les acides gras, les esters gras, les alcools gras et leurs mélanges. D'un intérêt tout particulier sont les substances hydrophobes dont le point de fusion est supérieur à 60°C et, plus spécialement l'acide stéarique dont la pureté est supérieure à 90 % et l'acide béhénique.

Les charges minérales qui conviennent particulièrement bien sont des charges lamellaires telles que l'aluminium en paillettes et le talc ou les silices. Plus particulièrement efficaces sont les silices traitées hydrophobes.

Généralement, dans la composition de pré-enrobage, le polymère basique, éventuellement en association avec le polymère non hydrosoluble, représente 0,5 à 10 %, de préférence 0,8 à 6 % en poids du noyau et la charge minérale représente 5 à 15 %, de préférence 8 à 12 % en poids du noyau.

Généralement la couche de pré-enrobage a une épaisseur comprise entre 5 et 10 μm.

Lorsque le noyau a subi un traitement de surface, il est particulièrement avantageux d'utiliser une composition de pré-enrobage constituée d'un copolymère basique associé à une substance hydrophobe dont le point de fusion est supérieur à 60°C afin de limiter la quantité de polymère aminé basique. Généralement le rapport copolymère basique/substance hydrophobe est compris entre 5/95 et 50/50 et plus particulièrement entre 10/90 et 20/80.

La couche de sur-enrobage est généralement constituée d'un mélange de substances hydrophobes. Les substances hydrophobes sont choisies de telle manière que la couche externe d'enrobage présente une texture qui permet la diffusion ou la pénétration du milieu liquide extérieur. En plus d'une faible perméabilité à l'eau, les substances hydrophobes doivent avoir des propriétés mécaniques convenables telles que résistance élevée à la traction et à l'allongement et caractère filmogène.

Parmi les substances hydrophobes peuvent être citées les matières grasses, les paraffines, les cires naturelles (cire de Carnauba, cire d'abeilles), les cires synthétiques (cire de polyéthylène), les polymères tels que le polyéthylène, le polypropylène, les polybutènes, les polyisobutènes, les polypentènes, le polystyrène, le chlorure ou le fluorure de polyvinyle, le chlorure ou le fluorure de polyvinylidène, les polyphénylènes, les oxydes de polyphénylène, le polybutadiène, le polyisoprène ou le polychloroprène, le polyacétate de vinyle, les dérivés cellulosiques non hydrosolubles et les latex ; les substances hydrophobes peuvent être utilisées seules mais de préférence en mélange de façon à obtenir une couche externe ayant les propriétés mécaniques désirées.

L'épaisseur de la couche de sur-enrobage est liée à la viscosité de la composition hydrophobe à l'état fondu.

Pour obtenir un sur-enrobage mince, il est particulièrement intéressant que la viscosité à l'état fondu soit comprise entre 2 et 100 poises.

Lorsque la viscosité est trop faible, l'enrobage n'est pas satisfaisant. Lorsque la viscosité est trop élevée, la couche de sur-enrobage est trop épaisse et il se produit des phénomènes d'agglomération.

Il est particulièrement avantageux d'utiliser une composition de sur-enrobage constituée d'un polymère hydrophobe filmogène (tel que le polyéthylène), d'un diluant qui diminue la viscosité de la phase fondue (tel qu'une paraffine fondant au voisinage de 60°C) et d'un agent assurant la tenue mécanique qui présente une grande fluidité à l'état fondu et une grande dureté à l'état solide (tel que le Polywax 500 ou la cire de polyéthylène) conférant ainsi une bonne résistance mécanique et évitant le collage à la température d'utilisation.

Dans les compositions selon l'invention, la couche externe d'enrobage, dont l'épaisseur moyenne peut varier de 5 à 200 microns selon la taille du granulé à enrober, représente de 1 à 50 % du poids total de la composition.

Pour améliorer les propriétés des compositions selon l'invention, il peut être avantageux d'ajouter à la composition hydrophobe de sur-enrobage une charge minérale pulvérulente choisie parmi les silices pyrogénées ou précipitées. La teneur en charge minérale représente généralement de 0,1 à 30 % et, de préférence de 1 à 15 % en poids de la composition de sur-enrobage.

Selon la présente invention, les compositions peuvent contenir des adjuvants dont le rôle est de faciliter la mise en oeuvre des techniques de préparation de ces compositions ou d'améliorer les caractéristiques physico-chimiques. Il peut être avantageux d'ajouter des plastifiants (triacétine, propylèneglycol), des agents lubrifiants (stéarate de magnésium), des agents liants (polyvinylpyrrolidone, alcool polyvinylique, gélatine), des agents antistatiques (triglycérides à chaînes polyoxy méthylénées), des agents anti-mottants, des agents fongicides, des agents émulsifiants, des agents de compatibilisation, des sucres, des amidons ou des protéines. Ces dérivés adjuvants ne représentent généralement que quelques pourcents en poids de l'enrobage.

Les substances actives entrant dans les compositions selon l'invention sont des substances thérapeutiques ou nutritives diverses destinées à être administrées par voie orale à des ruminants, et plus particulièrement celles dont la solubilité dans l'eau est élevée et généralement supérieure à 100 g/litre. Parmi ces substances actives peut être plus particulièrement cité le chlorhydrate de lysine.

Les nouvelles compositions selon l'invention sont, de préférence, des granulés, généralement sphériques ou cylindriques, dont le diamètre moyen est compris entre 0,05 et 5 mm.

Les nouvelles compositions selon l'invention peuvent être préparées par application des techniques connues de granulation et d'enrobage.

Le traitement de surface du noyau est généralement réalisé par "spray-coating" d'une dispersion de la charge minérale dans une solution organique du liant.

Le pré-enrobage des granulés traités en surface peut être effectué selon les techniques habituelles d'enrobage telles que l'encapsulation en lit fluidisé, le trempage ou la coacervation.

Pour effectuer le sur-enrobage de la substance active pré-enrobée différentes techniques peuvent être utilisées.

Il est possible d'effectuer le sur-enrobage en lit fluidisé, par trempage, par adsorption en milieu liquide ou par coacervation.

Il est également possible d'effectuer l'enrobage par la substance hydrophobe fondue ou en solution en projetant une suspension de la substance active pré-enrobée, dans la substance fondue ou en solution dans un solvant organique convenable dans lequel la substance active pré-enrobée n'est pas soluble sur un disque plat ou concave comportant éventuellement des rainures tournant à une vitesse déterminée et chauffé par de l'air chaud généralement à une température supérieure de 20°C à la température de solidification de la composition hydrophobe. Généralement la substance active pré-enrobée est dispersée dans 2 fois son poids de composition hydrophobe.

L'excès de composition hydrophobe forme des petites particules qui tombent à proximité du disque alors que les particules de substance active enrobée sont éjectées plus loin. Il en résulte que la séparation des particules de substance active enrobée et des particules de composition hydrophobe se fait systématiquement au cours de la mise en oeuvre du procédé. Par ailleurs, l'excès de composition hydrophobe peut être recyclé.

Les exemples suivants montrent comment l'invention peut être mise en pratique.

Le traitement de surface est effectué par spray-coating d'une dispersion de la charge minérale dans une solution du liant dans un solvant organique [chlorure de méthylène-méthanol (50-50 en volumes)].

Le pré-enrobage est effectué par traitement des granulés éventuellement pré-traités en lit fluidisé par une solution dans un solvant organique (tétrahydrofuranne, chlorure de méthylène) de la composition de pré-enrobage sensible aux variations du pH contenant éventuellement une charge minérale dispersée.

Le sur-enrobage est effectué en dispersant 50 g de granulés pré-enrobés dans 100 g de composition hydrophobe fondue contenant éventuellement une charge minérale dispersée, puis en coulant la dispersion au centre d'un disque horizontal tournant à une vitesse voisine de 1500 tours/minute et dont la température est maintenue au-dessus de la température de fusion de la composition hydrophobe. Les granulés sont éjectés du disque, la pellicule se solidifiant par refroidissement spontané au cours de la trajectoire de chute.

Le relargage de la substance active contenue dans les granulés obtenus est examiné, dans des conditions déterminées, en agitant une quantité connue de granulés dans un milieu tamponné maintenu à pH constant à une température de 40°C. On compare les vitesses de relargage d'un échantillon soumis à différents pH.

La résistance au rumen est déterminée de la manière suivante :
Environ 200 mg d'échantillon à tester sont pesés exactement et sont introduits dans un sachet nylon dont le vide de maille est 300 x 300 μm. Les sachets sont fermés par thermo-scellage et mis à incuber dans le rumen de brebis fistulées. Après 48 heures, les sachets sont récupérés, rincés et le principe actif restant est dosé.

EXEMPLE 1 (exemple comparatif)

On effectue un pré-enrobage de méthionine sous forme de granulés dont le diamètre moyen est compris entre 0,50 et 0,63 mm par du copolymère vinyl-2 pryidine-styrène de façon que l'épaisseur de la couche de pré-enrobage soit voisine de 8 μm.

Les granulés ainsi obtenus sont enrobés par une composition hydrophobe constituée de polyéthylène de faible poids moléculaire (20), de cire de polyéthylène (50) et de paraffine fondant à 60°C (30).

On obtient ainsi des granulés dont le titre en méthionine est voisin de 60 %.

Les résultats des essais de relargage sont donnés dans le tableau 1.

EXEMPLE 2 (exemple comparatif)

On opère comme dans l'exemple 1 mais en remplaçant la méthionine par du chlorhydrate de lysine.

Les résultats des essais de relargage sont donnés dans le tableau 1.

## TABLEAU 1

| Exemple | Titre | % relargué à 40°C X | | pH = 2 | % de résistance au rumen |
|---|---|---|---|---|---|
| | | pH = 6 | | | |
| | | après 5 heures | après 24 heures | après 2 heures | |
| 1 | 60 | 2 | 2 | 96 | 90 ± 9 |
| 2 | 61 | 100 | | 100 | 0 |

X en présence de 0,5 % de Tween 80

EXEMPLES 3 à 14

On utilise du chlorhydrate de lysine contenant 15 % d'Avicel (cellulose microcristalline) sous forme de granulés dont le diamètre moyen est compris entre 0,8 et 1 mm.

Les granulés sont pré-traités par de la zéine (10 % en poids des granulés) contenant des charges minérales.

Le pré-enrobage est réalisé par un copolymère vinyl-2 pyridine-styrène (70-30) de telle manière que le pré-enrobage représente 4 % du poids du noyau.

Le sur-enrobage est réalisé par une composition constituée de polyéthylène de faible masse moléculaire (20), cire de polyéthyléne (50), paraffine (30) (composition P) contenant éventuellement de l'Aerosil 300 (4) (composition Q).

Les résultats des essais de relargage et de résistance au rumen sont donnés dans le tableau 2.

TABLEAU 2

| Exemple | Traitement de surface | Sur-enrobage | Titre en lysine, HCl % | % de relargage à 40°C * | | | % de résistance au rumen (48 h) |
|---|---|---|---|---|---|---|---|
| | | | | à pH = 6 | | à pH = 2 | |
| | | | | après 5 heures | après 24 heures | après 2 heures | |
| 3 (comparatif) | - | P | 54 | 97 | 100 | 100 | 24 ± 2 |
| 4 (comparatif) | | Q | 57 | 51 | 76 | 93 | 29 ± 2 |
| 5 | zéine (10 %) | P | 50 | 34 | 78 | 100 | 46 ± 3 |
| 6 | zéine (10 %) | Q | 50 | 14 | 58 | 100 | 73 ± 3 |
| 7 | zéine (10 %) CaCO₃ (10 %) | P | 52 | 17 | 71 | 100 | 49 ± 4 |
| 8 | zéine (10 %) CaCO₃ (5 %) | P | 55 | 33 | 68 | 93 | 84 ± 2 |
| 9 | zéine (10 %) CaCO₃ (5 %) | Q | 48 | 5 | 9 | 82 | 60 ± 2 |
| 10 | zéine (10 %) kaolin (10 %) | P | 49 | | 39 | 97 | 91 ± 3 |
| 11 | zéine (10 %) kaolin (10 %) | Q | 47 | | 14 | 100 | 80 ± 4 |
| 12 | zéine (10 %) Aerosil 300 (10 %) | P | 50 | | 15 | 95 | 96 ± 4 |
| 13 | zéine (10 %) Aerosil 300 (10 %) | Q | 45 | | 7 | 85 | |
| 14 | zéine (10 %) lévilite (10 %) | P | 50 | 8 | 20 | 100 | 81,5 ± 7,7 |

* en présence de 0,5 % de Tween 80

EXEMPLES 15 à 16

On opère comme dans les exemples 3 à 14, en utilisant du chlorhydrate de lysine contenant 15 % d'Avi- cel (cellulose microcristalline) sous forme de granulés dont le diamètre moyen est compris entre 0,8 et 1 mm.

Le traitement de surface est effectué par une composition d'éthylcellulose (10 % en poids du noyau) et d'Aerosil 300 (10 % en poids du noyau).

Le pré-enrobage est effectué par une composition sensible aux variations du pH constituée de copolymère vinyl-2 pyridine-styrène (70-30) et d'acide stéarique (20-80 en poids).

Le sur-enrobage est effectué soit avec la composition P soit avec la composition Q décrites précédemment.

Les résultats des essais de relargage et de résistance au rumen sont donnés dans le tableau 3.

### TABLEAU 3

| Exemple | Sur-enrobage | Titre en lysine, HCl % | % de relargage à 40°C à pH = 6 X après 5 h | % de relargage à 40°C à pH = 6 X après 24 h | % de résistance au rumen (48 h) |
|---------|--------------|------------------------|---------|----------|-----|
| 15 | P | 49,8 | 12 | 21 | 89,6 ± 2,8 |
| 16 | Q | 50,2 | 12 | 24 | 78,0 ± 0,5 |

X en présence de 0,5 % de Tween 80.

### EXEMPLES 17 à 26

On utilise du chlorhydrate de lysine contenant 15 % d'Avicel (cellulose microscristalline) sous forme de granulés dont le diamètre moyen est compris entre 0,8 et 1 mm et qui ne sont pas pré-traités.

Le pré-enrobage est effectué par un copolymère vinyl-2 pyridine-styrène (V2P-S) (70-30) contenant en dispersion une charge minérale.

Le sur-enrobage est effectué soit avec la composition P soit avec la composition Q décrites précédemment.

Les résultats des essais de relargage et de résistance au rumen sont rassemblés dans le tableau 4.

TABLEAU 4

| Exemples | Pré-enrobage | Sur-enrobage | Titre en lysine, HCl % | % de relargage à 20°C X | | | % résistance au rumen (48 h) |
|---|---|---|---|---|---|---|---|
| | | | | à pH=6 | | à pH=2 | |
| | | | | après 8 h | après 24 h | après 2 h | |
| 17 | V2P-2 (4 %) | P | 59 | | 96 | 98 | 8 ± 1 |
| 18 | V2P-S (4 %) | Q | 57 | | 51 | | 33 ± 2 |
| 19 | V2P-S (4 %) Al paillettes (6%) | P | 53 | 10 | 18 | 99 | 77 ± 6 |
| 20 | V2P-S (4 %) Al paillettes (6%) | Q | 48 | 15 | 21 | 67 | |
| 21 | V2P-S (4 %) Aerosil 300 (10 %) | P | 55 | 12 | 20 | 64 | 80 ± 2 |
| 22 | V2P-S (4 %) Aerosil 300 (10 %) | Q | 47 | 7 | 9 | 34 | 80 ± 2 |
| 23 | V2P-S (4 %) Aerosil 300 (5 %) | P | 52 | 20 | 25 | 91 | 71 |
| 24 | V2P-S (4 %) Aerosil 300 (5 %) | Q | 47 | 27 | 26 | 86 | 71 ± 5 |
| 25 | V2P-S (4 %) Sipernat 17 (10 %) | P | 50 | 20 | 29 | 100 | 78,0 ± 2,8 |
| 26 | V2P-S (4 %) Aerosil R 972 (10 %) | P | 49 | 19 | 37 | | 60,8 ± 5,7 |
| 27 | V2P-S (4 %) talc (10 %) | P | 52 | 18 | 37 | 100 | 61,7 ± 7,2 |

X en présence de 0,5 % de Tween 80.

EXEMPLES 28 à 30

On utilise du chlorhydrate de lysine contenant 15 % d'Avicel (cellulose microcristalline) sous forme de granulés dont le diamètre moyen est compris entre 0,8 et 1 mm.

Les granulés sont traités en surface par une composition contenant 10 % en poids du noyau de zéine et 10 % en poids du noyau de kaolin.

La composition de pré-enrobage est constituée par du copolymère vinyl-2 pyridine-styrène (70-30). Le pré-enrobage représente environ 4 % du poids du noyau (épaisseur voisine de 7 μm).

Le sur-enrobage est réalisé par une composition constituée de polyéthylène de faible masse moléculaire (20), de Polywax 500 (50), de paraffine (30) et d'une charge minérale. La teneur en charge minérale de la composition de sur-enrobage est déterminée de telle manière que la viscosité du mélange fondu soit la même dans tous les essais.

Les résultats des essais de relargage et de résistance au rumen sont rassemblés dans le tableau 5.

TABLEAU 5

| Exemple | Charge minérale | Teneur pour 100 parties de composition hydrophobe | Titre en lysine, HCl % | % de relargage à 40°C à pH = 6 après 5 h | après 24 h | % de résistance au rumen (48 h) |
|---|---|---|---|---|---|---|
| 28 | – | – | 53 | 21 | 78 | 43,0 ± 9,8 |
| 29 | Aerosil 300 | 4 | 49 | 6 | 21 | 87,9 ± 6,1 |
| 30 | Lévilite | 12 | 50 | 3 | 10 | 80,3 ± 1,3 |

Pour la composition de l'exemple 30, le pourcentage de relargage à pH=2 est 92 après 2 heures à 40°C.

## Revendications

**REVENDICATIONS POUR LES ETATS CONTRACTANTS : AT, BE, CH, DE, FR, GB, IT, LI, LU, NL, SE**

1 - Composition pour l'alimentation des ruminants contenant une substance biologiquement active hydrosoluble, qui est stable dans un milieu dont le pH est égal ou supérieur à 5,5 et qui permet la libération de la substance active dans un milieu dont le pH est égal ou inférieur à 3,5 caractérisée en ce qu'elle comprend :

1) un noyau constitué d'une substance active hydrosoluble ou d'une composition contenant une substance active hydrosoluble ayant éventuellement subi un traitement de surface,

2) une première couche d'enrobage constituée d'une substance ou d'une composition sensible aux variations du pH éventuellement associée à une charge minérale, et

3) une deuxième couche d'enrobage constituée d'une substance ou d'une composition hydrophobe éventuellement associée à une charge minérale, étant entendu que

- lorsque le noyau a subi un traitement de surface, la couche de pré-enrobage et la couche de sur-enrobage ne contiennent pas nécessairement de charge minérale associée, et que

- lorsque le noyau n'a pas subi de traitement de surface, la couche de pré-enrobage et éventuellement la couche de sur-enrobage contiennent une charge minérale associée.

2 - Composition selon la revendication 1 caractérisée en ce que le granulé est traité en surface au moyen d'une composition constituée d'un liant de nature hydrophobe contenant éventuellement une charge minérale de nature hydrophile.

3 - Composition selon la revendication 2 caractérisée en ce que le liant est choisi parmi la zéine, l'éthylcellulose ou l'acétobutyrate de cellulose ou parmi les copolymères basiques sensibles aux variations du pH éventuellement en combinaison avec une substance hydrophobe.

4 - Composition selon la revendication 2 caractérisée en ce que la charge minérale est choisie parmi les silices pyrogénées ou précipitées, le carbonate de calcium ou le kaolin.

5 - Composition selon la revendication 1 caractérisée en ce que la première couche ou couche de pré-enrobage est constituée d'un polymère aminé basique filmogène sensible aux variations du pH éventuellement en association avec un polymère non hydrosoluble et/ou une substance hydrophobe, dans laquelle est éventuellement dispersée une charge minérale lamellaire ou une silice.

6 - Composition selon la revendication 5 caractérisée en ce que le polymère aminé basique est choisi parmi les dérivés aminés de la cellulose, les polymères ou copolymères des dérivés aminés des acides acryliques, méthacryliques ou crotoniques et les polymères ou copolymères du styrène ou de l'acrylonitrile avec les isomères ou les dérivés de la vinylpyridine.

7 - Composition selon la revendication 6 caractérisé en ce que le polymère aminé basique est choisi parmi les copolymères du styrène avec la vinyl-2 pyridine, la vinyl-4 pyridine ou la méthyl-2 vinyl-5 pyridine.

8 - Composition selon la revendication 5 caractérisé en ce que le polymère non hydrosoluble est choisi parmi l'éthylcellulose, l'acétobutyrate de cellulose ou le propionate de cellulose ou la zéine.

9 - Composition selon la revendication 5 caractérisée en ce que la substance hydrophobe est choisie parmi les acides gras, les esters gras, les alcools gras et leurs mélanges.

10 - Composition selon la revendication 9 caractérisée en ce que la substance hydrophobe est l'acide stéarique dont la pureté est supérieure à 90 %.

11 - Composition selon la revendication 5 caractérisée en ce que la charge minérale est choisie parmi l'aluminium en paillettes, le talc et les silices.

12 - Composition selon la revendication 11 caractérisée en ce que les silices sont choisies parmi les silices traitées hydrophobes.

13 - Composition selon la revendication 1 caractérisée en ce que la deuxième couche d'enrobage ou couche de sur-enrobage est constituée de substances hydrophobes choisies parmi les matières grasses, les paraffines, les cires naturelles ou synthétiques, les polymères tels que le polyéthylène, le polypropylène, les polybutènes, les polyisobutènes, les polypentènes, le polystyrène, le chlorure ou le fluorure de polyvinyle, le chlorure ou le fluorure de polyvinylidène, les polyphénylènes, les oxydes de polyphénylène, le polybutadiène, le polyisoprène ou le polychloroprène, le polyacétate de vinyle, les dérivés cellulosiques non hydrosolubles et les latex, utilisées seules ou en mélanges, éventuellement associées à une charge minérale.

14 - Composition selon la revendication 13 caractérisée en ce que la substance hydrophobe est constituée d'un mélange de polyéthylène de faible masse moléculaire, d'une cire de polyéthylène et d'une paraffine.

15 - Composition selon la revendication 12 caractérisée en ce que la charge minérale est choisie parmi les silices pyrogénées ou précipitées.

16 - Composition selon la revendication 1 caractérisée en ce que la substance biologiquement active est choisie parmi les substances thérapeutiques ou nutritives hydrosolubles administrées par voie orale aux ruminants.

17 - Composition selon la revendication 1 caractérisée en ce que la substance biologiquement active est le chlorhydrate de lysine.

18 - Procédé de préparation d'une composition selon la revendication 1 caractérisé en ce que l'on enrobe un granulé contenant la substance biologiquement active, ayant éventuellement subi un prétraitement de surface, par une composition selon l'une des revendications 5 à 12 par une technique d'encapsulation en lit fluidisé, par trempage ou par coacervation, puis enrobe le granulé pré-enrobé ainsi obtenu par une composition selon l'une des revendications 13 à 15 par encapsulation en lit fluidisé, par trempage, par adsorption en milieu liquide ou par coacervation.

19 - Procédé selon la revendication 18 caractérisé en ce que le sur-enrobage est effectué en projetant une dispersion du granulé enrobé dans la substance hydrophobe fondue ou en solution dans un solvant organique convenable sur un disque horizontal tournant à une vitesse déterminée.

20 - Procédé selon la revendication 18 caractérisé en ce que le traitement de surface du granulé est effectué par spray-coating d'une composition selon l'une des revendications 2 à 4.

## REVENDICATIONS POUR L'ETAT CONTRACTANT SUIVANT : ES.

1 - Procédé de préparation d'une composition pour l'alimentation des ruminants contenant une substance biologiquement active hydrosoluble, qui est stable dans un milieu dont le pH est égal ou supérieur à 5,5 et qui permet la libération de la substance active dans un milieu dont le pH est égal ou inférieur à 3,5 caractérisé en ce que :

1) l'on traite en surface, par spray-coating, un noyau constitué d'une substance active hydrosoluble ou une composition contenant une substance active hydrosoluble par une composition constituée d'un liant hydrophobe contenant éventuellement une charge minérale hydrophile,

2) enrobe les granulés pré-traités ainsi obtenus par une technique d'encapsulation en lit fluidisé, de trempage ou de coacervation au moyen d'une composition sensible aux variations du pH éventuellement associée à une charge minérale, puis,

3) enrobe les granulés ainsi obtenus par une technique d'encapsulation en lit fluidisé, de trempage, d'absorption en milieu liquide ou de coacervation par une composition hydrophobe éventuellement associée à une charge minérale.

2 - Procédé selon la revendication 1 caractérisé en ce que le traitement de surface par spray-coating est effectué au moyen d'une composition dans laquelle le liant de nature hydrophobe est choisi parmi la zéine, l'éthylcellulose ou l'acétobutyrate de cellulose ou parmi les copolymères basiques sensibles aux variations du pH éventuellement en combinaison avec une substance hydrophobe et la charge minérale éventuellement associée est choisie parmi les silices pyrogénées ou précipitées, le carbonate de calcium ou le koalin.

3 - Procédé selon la revendication 1 caractérisé en ce que l'enrobage des granulés pré-traités est effectué au moyen d'une composition constituée d'un polymère aminé basique filmogène sensible aux variations du pH éventuellement en association avec un polymère non hydrosoluble et/ou une substance hydrophobe

dans laquelle est éventuellement dispersée une charge minérale lamellaire ou une silice.

4 - Procédé selon la revendication 3 caractérisé en ce que le polymère aminé basique est choisi parmi les dérivés aminés de la cellulose, les polymères ou copolymères des dérivés aminés des acides acryliques, méthacryliques ou crotoniques et les copolymères du styrène ou de l'acrylonitrile avec les isomères ou les dérivés de la vinylpyridine.

5 - Procédé selon la revendication 4 caractérisé en ce que le polymère aminé basique est choisi par les copolymères du styrène avec la vinyl-2 pyridine, la vinyl-4 pyridine ou la méthyl-2 vinyl-5 pyridine.

6 - Procédé selon la revendication 3 caractérisé en ce que le polymère non hydrosoluble est choisi parmi l'éthylcellulose, l'acétobutyrate de cellulose ou le propionate de cellulose ou la zéine.

7 - Procédé selon la revendication 3 caractérisée en ce que la substance hydrophobe est choisie parmi les acides gras, les esters gras, les alcools gras et leurs mélanges.

8 - Procédé selon la revendication 7 caractérisée en ce que la substance hydrophobe est l'acide stéarique dont la pureté est supérieure à 90 %.

9 - Procédé selon la revendication 3 caractérisé en ce que la charge minérale est choisie parmi l'aluminium en paillettes, le talc et les silices traitées hyrdophobes.

10 - Procédé selon la revendication 1 caractérisé en ce que l'enrobage externe est effectué au moyen d'une composition constituée de substances hydrophobes choisies parmi les matières grasses, les paraffines, les cires naturelles ou synthétiques, les polymères tels que le polyéthylène, le polypropylène, les polybutènes, les polyisobutines, les polypentènes, le polystyrène, le chlorure ou le fluorure de polyvinyle, le chlorure ou le fluorure de polyvinylidène, les polyphénylènes, les oxydes de polyphénylène, le polybutadiène, le polyisoprène ou le polychloroprène, le polyacétate de vinyle, les dérivés cellulosiques non hydrosolubles et les latex, utilisées seules ou en mélanges, éventuellement associées à une charge minérale.

11 - Procédé selon la revendication 10 caractérisée en ce que la substance hydrophobe est constituée d'une mélange de polyéthylène de faible masse moléculaire, d'une cire de polyéthylène et d'une paraffine.

12 - Procédé selon la revendication 10 caractérisée en ce que la charge minérale est choisie parmi les silices pyrogénées ou précipitées.

13 - Procédé selon la revendication 1 caractérisée en ce que la substance biologiquement active est choisie parmi les substances thérapeutiques ou nutritives hydrosolubles administrées par voie orale aux ruminants.

14 - Procédé selon la revendication 13 caractérisée en ce que la substance biologiquement active est le chlorhydrate de lysine.


## Ansprüche

## PATENTANSPRÜCHE FÜR DIE VERTRAGSSTAAT : AT, BE, CH, DE, FR, GB, IT, LI, LU, NL, SE

1. Eine wasserlösliche, biologisch aktive Substanz enthaltendes Futtermittel für Wiederkäuer, das in einem Milieu mit einem pH gleich oder größer 5,5 stabil ist und das die Freisetzung der aktiven Substanz in einem Milieu mit einem pH gleich oder kleiner 3,5 zuläßt, dadurch gekennzeichnet, daß es umfaßt :
1) einen Kern, bestehend aus einer wasserlöslichen aktiven Substanz oder einer eine wasserlösliche aktive Substanz enthaltenden Masse, die gegebenenfalls oberflächenbebandelt wurde,
2) einer ersten Umhüllungsschicht, bestehend aus einer gegenüber pH-Änderungen empfindlichen Substanz, gegebenenfalls in Kombination mit einem anorganischen Füllstoff,
3) einer zweiten Umhüllungsschicht, bestehend aus einer hydrophoben Substanz oder Masse, gegebenenfalls kombiniert mit einem anorganischen Füllstoff, wobei selbstverständlich
- wenn der Kern oberflächenbehandelt worden ist, die Vor-Umhüllungsschicht und die Nach-Umhüllungsschicht nicht unbedingt einen kombinierten anorganischen Füllstoff enthalten und
wenn der Kern nicht oberflächenbehandelt worden ist, die Vor-Umhüllungsschicht und gegebenenfalls die Nach-umhüllungsschicht einen kombinierten anorganischen Füllstoff enthalten.

2. Mittel nach Anspruch 1, dadurch gekennzeichnet, daß das Granulat mit einer Masse oberflächenbehandelt wird, die aus einem hydrophoben Bindemittel, das gegebenenfalls einen hydrophilen anorganischen Füllstoff enthält, besteht.

3. Mittel nach Anspruch 2, dadurch gekennzeichnet, daß das Bindemittel ausgewählt ist aus Zein, Äthylcellulose oder Celluloseacetobutyrat oder den gegenüber pH-Änderungen empfindlichen basischen Copolymeren, gegebenenfalls in Kombination mit einer hydrophoben Substanz.

4. Mittel nach Anspruch 2, dadurch gekennzeichnet, daß der anorganische Füllstoff gewählt ist aus den pyrogenen oder gefällten Siliciumdioxiden, Calciumcarbonat oder Kaolin.

5. Mittel nach Anspruch 1, dadurch gekennzeichnet, daß die erste Schicht oder Vor-Umhüllungsschicht aus einem gegenüber pH-Änderungen empfindlichen, filmbildenden basischen Aminpolymer, gegebenenfalls in Kombination mit einem nicht wasserlöslichen Polymer und/oder einer hydrophoben Substanz besteht, worin gegebenenfalls ein plättchenförmiger anorganischer Füllstoff oder Siliciumdioxid dispergiert ist.

6. Mittel nach Anspruch 5, dadurch gekennzeichnet, daß das basische Aminpolymer ausgewählt ist aus den Aminderivaten von Cellulose, den Polymeren oder Copolymeren von Aminderivaten von Acryl-, Methacryl- oder Crotonsäure und den Polymeren oder Copolymeren von Styrol oder Acrylnitril mit den Isomeren oder den Derivaten von Vinylpyridin.

7. Mittel nach Anspruch 6, dadurch gekennzeichnet, daß das basische Aminpolymer ausgewählt ist aus den Copolymeren von Styrol mit 2-Vinylpyridin, 4-vinylpyridin oder 2-Methyl-5-vinylpyridin.

8. Mittel nach Anspruch 5, dadurch gekennzeichnet, daß das wasserunlösliche Polymer ausgewählt ist aus Äthylcellulose Celluloseacetobutyrat oder Cellulosepropionat oder Zein.

9. Mittel nach Anspruch 5, dadurch gekennzeichnet, daß die hydrophobe Substanz ausgewählt ist aus den Fettsäuren, den Fettestern, den Fettalkoholen und ihren Mischungen.

10. Mittel nach Anspruch 9, dadurch gekennzeichnet, daß die hydrophobe Substanz Stearinsäure mit einer Reinheit größer als 90 % ist.

11. Mittel nach Anspruch 5, dadurch gekennzeichnet, daß der anorganische Füllstoff ausgewählt ist aus Aluminiumplättchen, Talkum und den Siliciumdioxiden.

12. Mittel nach Anspruch 11, dadurch gekennzeichnet, daß die Siliciumdioxide ausgewählt sind aus den hydrophoben behandelten Siliciumdioxiden,

13. Mittel nach Anspruch 1, dadurch gekennzeichnet, daß die zweite Umhüllungsschicht oder Nach-Umhüllungsschicht aus hydrophoben Substanzen besteht, ausgewählt aus den Fetten, den Paraffinen, den natürlichen oder synthetischen Wachsen, den Polymeren wie Polyäthylen, Polypropylen, Polybutenen, Polyisobutenen, Polypentenen, Polystyrol, Polyvinylchlorid oder -fluorid, Polyvinylidenchlorid oder -fluorid, den Polyphenylenen, den Polyphenylenoxiden, Polybutadien, Polyisopren oder Polychloropren, Polyvinylacetat, den wasserunlöslichen Cellulosederivaten und den Latex, allein oder in Mischungen, gegebenenfalls zusammen mit einem anorganischen Füllstoff.

14. Mittel nach Anspruch 13, dadurch gekennzeichnet, daß die hydrophobe Substanz aus einer Mischung von Polyäthylen geringer Molekularmasse, einem Polyäthylenwachs und einem Paraffin besteht.

15. Mittel nach Anspruch 12, dadurch gekennzeichnet, daß die anorganische Charge gewählt ist aus den pyrogenen oder gefällten Siliciumdioxiden.

16. Mittel nach Anspruch 1, dadurch gekennzeichnet, daß die biologisch aktive Substanz ausgewählt ist aus den an Wiederkäuer oral verabreichten, wasserlöslichen Heil- oder Nährmitteln.

17. Mittel nach Anspruch 1, dadurch gekennzeichnet, daß die biologisch aktive Substanz Lysinchlorhydrat ist.

18. Verfahren zur Herstellung eines Mittels nach Anspruch 1, dadurch gekennzeichnet, daß man ein die biologisch aktive Substanz enthaltendes Granulat, dessen Oberfläche gegebenenfalls vorbehandelt worden ist, mit einer Masse nach einem der Ansprüche 5 bis 12 nach einer Verkapselungstechnik im Wirbelschichtbett, durch Tauchen oder Koazervieren umüllt und dann das so erhaltene vorumhüllte Granulat mit einer Masse nach einem der Ansprüche 13 bis 15 durch verkapselung im Wirbelschichtbett, durch Tauchen, durch Adsorption in flüssigem Milieu oder durch Koazervieren umhüllt.

19. Verfahren nach Anspruch 18, dadurch gekennzeichnet, daß die Nach-Umhüllung ausgeführt wird, indem man eine Dispersion des umhüllten Granulats in die geschmolzene oder in einem geeigneten organischen Lösungsmittel gelöste hydrophobe Substanz auf einer mit bestimmter Geschwindigkeit rotierenden horizontalen Scheibe sprüht.

20. Verfahren nach Anspruch 18, dadurch gekennzeichnet, daß die Oberflächenbehandlung des Granulats durch Sprühauftrag einer Masse nach einem der Ansprüchen 2 bis 4 ausgeführt wird.

## PATENTANSPRUCHE FUR DEN VERTRAGSSTAAT : ES

1. Verfahren zur Herstellung eines eine wasserlösliche, biologisch aktive Substanz enthaltenden Futtermittels für Wiederkäuer, das in einem Milieu mit einem pH gleich oder größer 5,5 stabil ist und das die Freisetzung der aktiven Substanz in einem Milieu mit einem pH gleich oder kleiner 3,5 zuläßt, dadurch gekennzeichnet, daß man

1) einen Kern, bestehend aus einer wasserlöslichen aktiven Substanz oder einer eine wasserlösliche aktive Substanz enthaltenden Masse, mit einem Mittel, bestehend aus einem hydrophoben Bindemittel, das gege-

EP 0 268 533 B1

benenfalls einen hydrophilen anorganischen Füllstoff enthält, durch Aufspritzen oberflächenbehandelt,

2) die so erhaltenen vorbehandelten Granulate durch Verkapselung im Wirbelschichtbett, durch Tauchen oder durch Koazervieren mit einer gegenüber pH-Änderungen empfindlichen Substanz, gegebenenfalls in Kombination mit einem anorganischen Füllstoff, umhüllt,

3) die so erhaltenen Granulate durch Verkapselung im Wirbelschichtbett, durch Tauchen, Absorption in flüssigem Milieu oder Koazervieren mit einer hydrophoben Masse, gegebenenfalls kombiniert mit einem anorganischen Füllstoff, umhüllt.

2. Verfahren nach Anspruch 1, dadurch gekennzeichnet, daß die Oberflächenbehandlung durch Aufspritzen mit einer Masse ausgeführt wird, in welcher das hydrophobe Bindemittel ausgewählt ist aus Zein, Äthylcellulose oder Celluloseacetobutyrat oder den gegenüber pH-Änderungen empfindlichen basischen Copolymeren, gegebenenfalls in Kombination mit einer hydrophoben Substanz und der gegebenenfalls mitverwendete anorganische Füllstoff gewählt ist aus den pyrogenen oder gefällten Siliciumdioxiden, Calciumcarbonat oder Kaolin.

3. Verfahren nach Anspruch I, dadurch gekennzeichnet, daß die Umhüllung der vorbehandelten Granulate mit einer Masse, bestehend aus einem gegenüber pH-Änderungen empfindlichen, filmbildenden basischen Aminpolymer, gegebenenfalls in Kombination mit einem nicht wasserlöslichen Polymer und/oder einer hydrophoben Substanz besteht, worin gegebenenfalls ein plättchenförmiger anorganischer Füllstoff oder Siliciumdioxid dispergiert ist, erfolgt.

4. Verfahren nach Anspruch 3, dadurch gekennzeichnet, daß das basische Aminpolymer ausgewählt ist aus den Aminderivaten von Cellulose, den Polymeren oder Copolymeren von Aminderivaten von Acryl-, Methacryl- oder Crotonsäure und den Polymeren oder Copolymeren von Styrol oder Acrylnitril mit den Isomeren oder den Derivaten von vinylpyridin.

5. Verfahren nach Anspruch 4, dadurch gekennzeichnet, daß das basische Aminpolymer ausgewählt ist aus den Copolymeren von Styrol mit 2-vinylpyridin, 4-vinylpyridin oder 2-Methyl-5-vinylpyridin.

6. Verfahren nach Anspruch 3, dadurch gekennzeichnet, daß das wasserunlösliche Polymer ausgewählt ist aus Äthylcellulose Celluloseacetobutyrat oder Cellulosepropionat oder Zein.

7. Verfahren nach Anspruch 3, dadurch gekennzeichnet, daß die hydrophobe Substanz ausgewählt ist aus den Fettsäuren, den Fettestern, den Fettalkoholen und ihren Mischungen.

8. Verfahren nach Anspruch 7, dadurch gekennzeichnet, daß die hydrophobe Substanz Stearinsäure mit einer Reinheit größer als 90 % ist.

9. Verfahren nach Anspruch 3, dadurch gekennzeichnet, daß der anorganische Füllstoff ausgewählt ist aus Aluminiumplättchen, Talkum und den Siliciumdioxiden.

10. Verfahren nach Anspruch 1, dadurch gekennzeichnet, daß die äußere Umhüllung mit einer Masse erfolgt, bestehend aus hydrophoben Substanzen, ausgewählt aus den Fetten, den Paraffinen, den natürlichen oder synthetischen Wachsen, den Polymeren wie Polyäthylen, Polypropylen, Polybutenen, Polyisobutenen, Polypentenen, Polystyrol, Polyvinylchlorid oder -fluorid, Polyvinylidenchlorid oder -fluorid, den Polyphenylenen, den polyphenylenoxiden, Polybutadien, Polyisopren oder Polychloropren, Polyvinylacetat, den wasserunlöslichen Cellulosederivaten und den Latex, allein oder in Mischungen, gegebenenfalls zusammen mit einem anorganischen Füllstoff.

11. Verfahren nach Anspruch 10, dadurch gekennzeichnet, daß die hydrophobe Substanz aus einer Mischung von Polyäthylen geringer Molekularmasse, einem Polyäthylenwachs und einem Paraffin besteht.

12. Mittel nach Anspruch 10, dadurch gekennzeichnet, daß die anorganische Charge gewählt ist aus den pyrogenen oder gefällten Siliciumdioxiden.

13. Verfahren nach Anspruch 1, dadurch gekennzeichnet, daß die biologisch aktive Substanz ausgewählt ist aus den an Wiederkäuer oral verabreichten, wasserlöslichen Heil- oder Nährmitteln.

14. Verfahren nach Anspruch 13, dadurch gekennzeichnet, daß die biologisch aktive Substanz Lysinchlorhydrat ist.

## Claims

### CLAIMS FOR THE CONTRACTING STATES : AT, BE, CH, DE, FR, GB, IT, LI, LU, NL, SE

1. A composition for feeding ruminants, containing a water-soluble biologically active substance which is stable in a medium whose pH is equal to or greater than 5.5 and which permits the release of the active substance in a medium whose pH is equal to or less than 3.5, which comprises :

1) a core consisting of a water-soluble active substance or of a composition containing a water-soluble active substance, which has optionally undergone a surface treatment,

13

2) a first coating layer consisting of a substance or composition that is sensitive to pH variations, optionally in combination with an inorganic filler, and

3) a second coating layer consisting of a hydrophobic substance or composition, optionally in combination with an inorganic filler, with the proviso that

- when the core has undergone a surface treatment, the precoating layer and the outer coating layer do not necessarily contain an inorganic filler in combination, and that

- when the core has not undergone a surface treatment, the precoating layer and optionally the outer coating layer contain an inorganic filler in combination.

2. The composition according to claim 1, in which the granule is surface-treated by means of a composition consisting of a binder that is hydrophobic in nature, optionally containing an inorganic filler that is hydrophilic in nature.

3. The composition according to claim 2, in which the binder is chosen from zein, ethylcellulose or cellulose acetobutyrate, or from basic copolymers sensitive to pH variations, optionally in combination with a hydrophobic substance.

4. The composition according to claim 2, in which the inorganic filler is chosen from pyrogenic or precipitated silicas, calcium carbonate or kaolin.

5. The composition according to claim 1, in which the first layer or precoating layer consists of a filmforming basic aminated polymer sensitive to pH variations, optionally in combination with a non-water-soluble polymer and/or a hydrophobic substance, in which a lamellar inorganic filler or a silica is optionally dispersed.

6. The composition according to claim 5, in which the basic aminated polymer is chosen from amino derivatives of cellulose, polymers or copolymers of amino derivatives of acrylic, methacrylic or crotonic acids and polymers or copolymers of styrene or acrylonitrile with isomers or derivatives of vinylpyridine.

7. The composition according to claim 6, in which the basic aminated polymer is chosen from the copolymers of styrene with 2-vinylpyridine, 4-vinylpyridine or 2-methyl-5-vinylpyridine.

8. The composition according to claim 5, in which the non-water-soluble polymer is chosen from ethylcellulose, cellulose acetobutyrate or cellulose propionate or zein.

9. The composition according to claim 5, in which the hydrophobic substance is chosen from fatty acids, fatty esters, fatty alcohols and mixtures thereof.

10. The composition according to claim 9, in which the hydrophobic substance is stearic acid whose purity is greater than 90%.

11. The composition according to claim 5, in which the inorganic filler is chosen from aluminium flakes, talc and silicas.

12. The composition according to claim 11, in which the silicas are chosen from hydrophobic treated silicas.

13. The composition according to claim 1, in which the second coating layer or outer coating layer consists of hydrophobic substances chosen from fats, paraffin waxes, natural or synthetic waxes, polymers such as polyethylene, polypropylene, polybutenes, polyisobutenes, polypentenes, polystyrene, polyvinyl chloride or fluoride, polyvinylidene chloride or fluoride, polyphenylenes, polyphenylene oxides, polybutadiene, polyisoprene or polychloroprene, polyvinyl acetate, non-water-soluble cellulose derivatives and latexes, used alone or as mixtures, optionally in combination with an inorganic filler.

14. The composition according to claim 13, in which the hydrophobic substance consists of a mixture of low molecular mass polyethylene, a polyethylene wax and a paraffin wax.

15. The composition according to claim 12, in which the inorganic filler is chosen from pyrogenic or precipitated silicas.

16. The composition according to claim 1, in which the biologically active substance is chosen from water-soluble therapeutic or nutrient substances administered orally to ruminants.

17. The composition according to claim 1, in which the biologically active substance is lysine hydrochloride.

18. A process for preparing a composition according to claim 1, in which a granule containing the biologically active substance, which has optionally undergone a surface pretreatment, is coated with a composition according to one of claims 5 to 12 by an encapsulation technique in a fluidized bed, by immersion or by coacervation, and the precoated granule thereby obtained is then coated with a composition according to one of claims 13 to 15 by encapsulation in a fluidized bed, by immersion, by adsorption in liquid medium or by coacervation.

19. The process according to claim 18, wherein the outer coating operation is performed by projecting a dispersion of the coated granule, in the molten hydrophobic substance or the hydrophobic substance dissolved in a suitable organic solvent, onto a horizontal disc rotating at a specified speed.

20. The process according to claim 18, wherein the surface treatment of the granule is performed by spraycoating with a composition according to one of claims 2 to 4.

## CLAIMS FOR THE CONTRACTING STATE : ES

1. A process for preparing a composition for feeding ruminants, containing a water-soluble biologically active substance which is stable in a medium whose pH is equal to or greater than 5.5 and which permits the release of the active substance in a medium whose pH is equal to or less than 3.5, in which :

1) a core consisting of a water-soluble active substance or a composition containing a water-soluble active substance is surface-treated, by spray-coating, with a composition consisting of a hydrophobic binder optionally containing a hydrophilic inorganic filler,

2) the pretreated granules thereby obtained are coated by a technique of encapsulation in a fluidized bed, of immersion or of coacervation, by means of a composition that is sensitive to pH variations, optionally in combination with an inorganic filler, then,

3) the granules thereby obtained are coated by a technique of encapsulation in a fluidized bed, of immersion, of absorption in liquid medium or of coacervation, with a hydrophobic composition optionally in combination with an inorganic filler.

2. The process according to claim 1, in which the surface treatment by spray-coating is performed by means of a composition in which the binder, that is hydrophobic in nature, is chosen from zein, ethylcellulose or cellulose acetobutyrate, or from basic copolymers sensitive to pH variations, optionally in combination with a hydrophobic substance and the inorganic filler, optionally in combination, is chosen from pyrogenic or precipitated silicas, calcium carbonate or kaolin.

3. The process according to claim 1, in which the coating of the pretreated granules is performed by means of a composition consisting of a film-forming basic aminated polymer sensitive to pH variations, optionally in combination with a non-water-soluble polymer and/or a hydrophobic substance, in which a lamellar inorganic filler or a silica is optionally dispersed.

4. The process according to claim 3, in which the basic aminated polymer is chosen from amino derivatives of cellulose, polymers or copolymers of amino derivatives of acrylic, methacrylic or crotonic acids and copolymers of styrene or acrylonitrile with isomers or derivatives of vinylpyridine.

5. The process according to claim 4, in which the basic aminated polymer is chosen from the copolymers of styrene with 2-vinylpyridine, 4-vinylpyridine or 2-methyl-5-vinylpyridine.

6. The process according to claim 3, in which the non-water-soluble polymer is chosen from ethylcellulose, cellulose acetobutyrate or cellulose propionate or zein.

7. The process according to claim 3, in which the hydrophobic substance is chosen from fatty acids, fatty esters, fatty alcohols and mixtures thereof.

8. The process according to claim 7, in which the hydrophobic substance is stearic acid whose purity is greater than 90%.

9. The process according to claim 3, in which the inorganic filler is chosen from aluminium flakes, talc and hydrophobic treated silicas.

10. The process according to claim 1, in which the outer coating is performed by means of a composition consisting of hydrophobic substances chosen from fats, paraffin waxes, natural or synthetic waxes, polymers such as polyethylene, polypropylene, polybutenes, polyisobutenes, polypentenes, polystyrene, polyvinyl chloride or fluoride, polyvinylidene chloride or fluoride, polyphenylenes, polyphenylene oxides, polybutadiene, polyisoprene or polychloroprene, polyvinyl acetate, non-water soluble cellulose derivatives and latexes, used alone or as mixtures, optionally in combination with an inorganic filler.

11. The process according to claim 10, in which the hydrophobic substance consists of a mixture of low molecular mass polyethylene, a polyethylene wax and a paraffin wax.

12. The process according to claim 10, in which the inorganic filler is chosen from pyrogenic or precipitated silicas.

13. The process according to claim 1, in which the biologically active substance is chosen from water-soluble therapeutic or nutrient substances administered orally to ruminants.

14. The process according to claim 13, in which the biologically active substance is lysine hydrochloride.